# FASCICULE DE BREVET EUROPEEN

(11) **EP 2 150 266 B1**
(45) Date de publication et mention de la délivrance du brevet: **11.06.2014**
(21) Numéro de dépôt: 08805494.5
(22) Date de dépôt: 23.04.2008
(51) Int. Cl.: A61K 38/08, A61K 38/45, A61K 8/64, A61P 17/00

(54) **COMPOSITION PHARMACEUTIQUE ET/OU COSMETIQUE CONTENANT UN PRINCIPE ACTIF ANTIOXYDANT ET ACTIVATEUR DE L'ENERGIE CELLULAIRE**
PHARMAZEUTISCHE UND/ODER KOSMETISCHE ZUSAMMENSETZUNG MIT EINEM WIRKSTOFF, DER ANTIOXIDATIONSMITTEL IST UND ZELLENERGIE AKTIVIERT
PHARMACEUTICAL AND/OR COSMETIC COMPOSITION CONTAINING AN ACTIVE INGREDIENT WHICH IS ANTIOXIDANT AND WHICH ACTIVATES CELL ENERGY

(30) Priorité: 27.04.2007 FR 0703063
(43) Date de publication de la demande: 10.02.2010
(73) Titulaire: Ashland Specialties France, 06410 Biot (FR)
(72) Inventeur: DAL FARRA, Claude, F-06650 Opio (FR); DOMLOGE, Nouha, F-06560 Valbonne (FR); BOTTO, Jean-Marie, F-06560 Valbonne (FR)
(74) Mandataire: Macquet, Christophe
(86) Numéro de dépôt international: PCT/FR2008/000575
(87) Numéro de publication internationale: WO 2008/145852

(56) Documents cités:
- WO-A-02/079430
- WO-A-03/077936
- WO-A-2004/043482
- WO-A-2005/097060
- US-B1- 6 410 827
- BAUZA E ET AL: "A NEW PEPTIDE THAT DISPLAYS AN UNCOUPLING-LIKE EFFECT ON THE MITOCHONDRIAL RESPIRATION PROCESS OF ADIPOCYTES AND REDUCES ADIPOCYTE LIPID SYNTHESIS" JOURNAL OF INVESTIGATIVE DERMATOLOGY, NEW YORK, NY, US, vol. 122, no. 3, mars 2004 (2004-03), page A71, XP009078779 ISSN: 0022-202X
- GONDRAN C ET AL: "A new synthetic peptide that exhibits interesting anti-aging effects" JOURNAL OF INVESTIGATIVE DERMATOLOGY, vol. 126, no. Suppl. 1, avril 2006 (2006-04), page 27, XP009093186 & 67TH ANNUAL MEETING OF THE SOCIETY-FOR-INVESTIGATIVE-DERMATOLOGY; PHILADELPHIA, PA, USA; MAY 03 -06, 2006 ISSN: 0022-202X
- DEL FARRA ET AL.: "Anti-aging effects observed in new synthetic peptide" J AM ACAD DERMATOL, février 2007 (2007-02), page AB25, XP002460912
- "An anti-aging effect on the lips and skin observed in in vivo studies on a new fibronectin-like peptide" J AM ACAD DERMATOL, février 2007 (2007-02), page AB88, XP002460913

### SEQUENCE LISTING

<110> société d'Extraction des Principes Actifs (ISP VINCIENCE)
<120> COMPOSITION PHARMACEUTIQUE ET/OU COSMETIQUE CONTENANT UN PRINCIPE ACTIF ANTIOXYDANT ET ACTIVATEUR DE L'ENERGIE CELLULAIRE
<130> Bv PCT 07-86
<150> FR0703063
   <151> 2007-04-27
<160> 2
<170> PatentIn version 3.3
<210> 1
   <211> 6
   <212> PRT
   <213> Artificial
<220>
   <223> Synthetic Peptide
<220>
   <221> MOD_RES
   <222> (6)..(6)
   <223> AMIDATION
<400> 1
   Ala Val Leu Ala Gly Asp
   1               5
<210> 2
   <211> 6
   <212> PRT
   <213> Artificial
<220>
   <223> Synthetic Peptide
<400> 2
   Ala Val Leu Ala Gly Asp
   1               5

## Description

La présente invention se situe dans le domaine cosmétique et pharmaceutique, et plus particulièrement dans le domaine de la dermatologie. La présente invention porte sur une <insert claim> et concerne une composition cosmétique ou pharmaceutique, et notamment dermatologique, comprenant, dans un milieu physiologiquement adapté, en tant que principe actif, certains peptides, utilisés seul ou en association avec au moins un autre principe actif. L'utilisation d'une composition capable d'augmenter l'énergie cellulaire et de protéger la peau des dommages oxydatifs et de protéger la peau et les phanères des agressions extérieures et de lutter contre le vieillissement cutané est également décrite. Le principe actif peut également être utilisé pour préparer des compositions pharmaceutiques destinées à prévenir ou lutter contre les pathologies liées à des processus d'oxydation ou encore, certaines pathologies du vieillissement.

Le terme « phanères » selon l'invention englobe l'ensemble des annexes kératiniques présentes à la surface du corps, en particulier les poils, les cils, les sourcils, les ongles et les cheveux.

La peau est un organe vital qui recouvre toute la surface du corps et assure des fonctions protectrices, sensitives, immunitaires, métaboliques ou encore thermorégulatrices. La peau, comme les autres organes, est soumise au vieillissement. Or, un des mécanismes majeurs impliqués dans les processus du vieillissement est l'accumulation de dommages oxydatifs dans des molécules essentielles telles que les lipides membranaires, les protéines, l'ADN et tout particulièrement l'ADN mitochondrial (ADNmt).

Les dommages oxydatifs sont provoqués par les radicaux libres, des espèces chimiquement instables et très réactionnelles, générées par le métabolisme intracellulaire ou les agressions extérieures. Parmi ces agressions extérieures, on peut citer : les rayonnements UV, les toxines, les polluants atmosphériques, les oxydants alimentaires. Dans la peau, on observe un vieillissement prématuré survenant dans les zones exposées aux rayonnements, caractérisé par des phénomènes d'altérations des macromolécules (péroxydation lipidique, carbonylation des protéines) touchant en particulier l'élastine, le collagène ou la fibronectine. On a également pu montrer un déclin progressif des fonctions mitochondriales avec l'âge, probablement lié à l'accumulation de mutations sur l'ADNmt (K. Singh, Ann. N.Y. Acad. Sci. 1019, 2004).

Une des conséquences importantes de l'accumulation des dommages oxydatifs est une réduction de la capacité de la cellule à produire de l'ATP (Porteous et al., Eur J Biochem 1998, 257(1):192-201). Ainsi, le phénomène du vieillissement cellulaire est en relation avec les dommages oxydatifs que subit la cellule mais aussi avec le processus de production d'énergie nécessaire à la cellule pour survivre.

L'organisme possède des mécanismes de défense, capables de piéger ou de transformer les radicaux libres (enzymes, glutathion, vitamines A et E, coenzyme Q10, etc.). Cependant, ces systèmes de défenses antioxydants s'avèrent souvent insuffisants devant les nombreux stress et agressions extérieures auxquels sont soumis les organismes et la peau en particulier.

Dans ce contexte, les propriétés antioxydantes du coenzyme Q10 apparaissent comme particulièrement intéressantes :

Le coenzyme Q10 (ou ubiquinone) est un coenzyme présent dans les complexes mitochondriaux impliqués dans la phosphorylation oxydative conduisant à la production d'ATP (Mitchell and al, 1976; Mitchell and al, 1990). L'autre propriété fondamentale du coenzyme Q10 est d'être un antioxydant, neutralisant les radicaux libres (Beyer et al 1990, Villalba et al 1997).

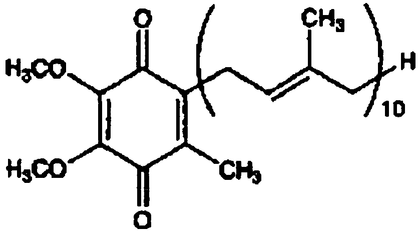

Le coenzyme Q10 est un dérivé benzoquinonique flanqué d'une longue chaîne latérale isoprénique composée le plus souvent de dix unités isoprénoïdes (d'où le nom de Coenzyme Q10). Ce coenzyme n'étant pas soluble dans l'eau, on ne le rencontre que dans des membranes lipidiques comme la membrane interne de la mitochondrie, où il peut diffuser librement parmi les phospholipides membranaires.

Le Coenzyme Q10 peut exister sous trois états d'oxydation : une forme réduite (CoQH2 ou UQH2), une forme oxydée (CoQ10), et une forme intermédiaire le radical ubisemiquinone (Q°).

Le coenzyme Q10 est présent dans la peau où il stimule les fonctions naturelles des cellules et agit en défenseur vis-à-vis des agressions extérieures.
La biosynthèse du coenzyme Q10 se fait à partir de la tyrosine pour le noyau quinone, et à partir du farnesyl pyrophosphate pour la chaîne latérale. L'enzyme responsable de cette dernière réaction, qui est une étape essentielle dans la biosynthèse du coenzyme Q10, est la transprényl transférase (ou polyprenyl transférase).

La recherche de composés pouvant stimuler la synthèse de coenzyme Q10, les synthèses énergétiques d'ATP et/ou protéger les cellules des dommages causés par les radicaux libres est une préoccupation de la recherche médicale et de la cosmétique. Il a ainsi été proposé des solutions par l'apport de substances d'origine peptidiques présentant des propriétés antioxydantes (WO2005097060, JP2006131626), mais aucune composition cosmétique ou pharmaceutique comprenant les peptides ou polypeptides de la présente invention n'a été décrite à ce jour.

Les inventeurs ont mis en évidence une activité thérapeutique, et plus particulièrement dermatologique et cosmétique, de peptides particuliers, décrits dans la présente invention. Il a notamment été mis en évidence que ces peptides, lorsqu'ils sont appliqués sur la peau, ont une forte activité protectrice vis-à-vis des dommages oxydatifs subis par la peau et favorisent de façon importante la synthèse d'ATP, ainsi que la synthèse ou l'activité de l'enzyme transprényl transférase et du coenzyme Q10. Ce nouveau principe actif, capable d'augmenter l'énergie cellulaire et de protéger la peau des dommages oxydatifs, permet ainsi d'ouvrir de nouvelles perspectives thérapeutiques et cosmétiques.

On entend par « principe actif capable d'augmenter l'énergie cellulaire et de protéger la peau des dommages oxydatifs» toute substance capable d'augmenter la synthèse d'ATP intracellulaire et de présenter des propriétés protectrices dans des cellules ou des tissus soumis à un stress oxydant d'origine physico-chimique ou environnementale.

L'objet de l'invention est décrit dans les revendications.

Une composition cosmétique ou pharmaceutique, et notamment dermatologique, comprenant, dans un milieu physiologiquement adapté, en tant que principe actif, des peptides capables d'augmenter l'énergie cellulaire et de protéger la peau des dommages oxydatifs, seul ou en association avec au moins un autre principe actif est décrite.

Le terme « peptide » désigne un enchaînement de deux ou plusieurs acides aminés liés entre eux par des liaisons peptidiques ou par des liaisons peptidiques modifiées.

Par « peptide », il faut entendre le peptide naturel ou synthétique de l'invention tel que décrit ci-dessus ou au moins l'un de ses fragments, qu'il soit obtenu par protéolyse ou de manière synthétique, ou encore tout peptide naturel ou synthétique dont la séquence est totalement ou partiellement constituée par la séquence du peptide selon l'invention. Selon la présente invention, le principe actif selon l'invention est composé d'au moins un peptide dont le nombre d'acides aminés est compris entre 5 et 13.

Le peptide possède une séquence qui répond à la formule générale (I)

R₁-(AA)ₙ- Ala- Val- Leu- Ala- Gly-(AA)ₚ-R₂

Dans laquelle
AA représente un acide aminé quelconque, ou un de ses dérivés, et n et p sont des nombres entiers compris entre 0 et 4.
R₁ représente la fonction amine primaire de l'acide aminé N-terminal, libre ou substituée par un groupement protecteur pouvant être choisi parmi un groupement acétyle, un groupement benzoyle, un groupement tosyle ou un groupement benzyloxycarbonyle.
R₂ représente la fonction hydroxyle de l'acide carboxyle de l'acide aminé C-terminal, libre ou substituée par un groupement protecteur pouvant être choisi parmi une chaîne alkyle de C₁ à C₂₀, ou un groupement NH₂, NHY ou NYY avec Y représentant une chaîne alkyle de C₁ à C₄.

Selon une méthode de réalisation de l'invention tout particulièrement préférée, le peptide biologiquement actif est de séquence SEQ ID n°1 ou de séquence SEQ ID n°2
(SEQ ID n°1) Ala-Val-Leu-Ala-Gly-Asp-NH2
(SEQ ID n°2) Ala-Val-Leu-Ala-Gly-Asp

Des formes homologues de ces séquences sont également décrites. Le terme « homologue » désigne, selon l'invention, toute séquence peptidique identique à au moins 50%, ou de préférence au moins 80%, et encore plus préférentiellement à au moins 90% de ladite séquence peptidique, choisie entre la séquence SEQ ID n° 1 et la séquence SEQ ID n° 2. Par « séquence peptidique identique à au moins X% », on entend désigner un pourcentage d'identité entre les résidus d'acides aminés des deux séquences à comparer, obtenu après l'alignement optimal des deux séquences. L'alignement optimal est obtenu à l'aide d'algorithmes d'homologies locales tels que ceux utilisés par les logiciels informatiques BLAST P ou T BLAST N disponibles sur le site NCBI.

Le terme « homologue » peut également désigner un peptide qui diffère de la séquence d'un peptide de séquence SEQ ID n° 1 ou SEQ ID n° 2 par la substitution d'acides aminés chimiquement équivalents, c'est-à-dire par la substitution d'un résidu par un autre possédant les mêmes caractéristiques. Ainsi, les substitutions classiques se font entre Ala, Val, Leu et Ile ; entre Ser et Thr ; entre les résidus acides Asp et Glu ; entre Asn et Gln ; et entre les résidus basiques Lys et Arg ; ou entre les résidus aromatiques Phe et Tyr.

Le terme « acide aminé » se réfère ici à tout acide organique naturel ou non naturel ayant la formule :

-NHR-CR-C(O)-O-

où chaque -R est indépendamment sélectionné entre un hydrogène et un groupement alkyl ayant entre 1 et 12 atomes de carbone. Préférentiellement, au moins un groupement -R de chaque acide aminé est un hydrogène. Par le terme « alkyl », on entend ici une chaîne carbonée pouvant être linéaire ou ramifiée, substituée (mono- ou poly-) ou non-substituée ; saturée, mono-saturée (une double ou triple liaison dans la chaîne) ou poly-insaturée (deux ou plusieurs doubles liaisons, deux ou plusieurs triples liaisons, une ou plusieurs doubles liaisons et une ou plusieurs triples liaisons dans la chaîne).

Par « peptide », il faut entendre le peptide naturel ou synthétique de l'invention tel que décrit ci-dessus ou au moins l'un de ses fragments, qu'il soit obtenu par protéolyse ou de manière synthétique, ou encore tout peptide naturel ou synthétique dont la séquence est totalement ou partiellement constituée par la séquence du peptide précédemment décrit.

De façon à améliorer la résistance à la dégradation, il peut être nécessaire d'utiliser une forme protégée du peptide selon l'invention. La forme de protection doit évidemment être une forme biologiquement compatible et doit être compatible avec une utilisation dans le domaine des cosmétiques ou de la pharmacie.

De nombreuses formes de protection biologiquement compatibles peuvent être envisagées. Ainsi, l'invention concerne une composition telle que définie précédemment, caractérisée par le fait que le peptide de formule générale (I) possède au moins un groupement fonctionnel protégé par un groupement protecteur, ce groupement protecteur étant soit une acylation ou une acétylation de l'extrémité amino-terminale, soit une amidation ou une estérification de l'extrémité carboxy-terminale, soit les deux. L'extrémité amino-terminale peut être protégée par un groupement acétyle, un groupement benzoyle, un groupement tosyle ou un groupement benzyloxycarbonyle. De préférence, on utilise une protection basée sur l'amidation de la fonction hydroxyle de l'extrémité carboxy-terminale par un groupement NYY avec Y représentant une chaîne alkyle de C₁ à C₄, ou l'estérification par un groupement alkyle. Il est également possible de protéger les deux extrémités du peptide.

Les dérivés de peptides concernent aussi les acides aminés et les peptides reliés entre eux par une liaison pseudo-peptidique. On entend par « liaison pseudo-peptidique », tous les types de liaisons susceptibles de remplacer les liaisons peptidiques « classiques ».

Dans le domaine des acides aminés, la géométrie des molécules est telle qu'elles peuvent théoriquement se présenter sous la forme d'isomères optiques différents. Il existe, en effet, une conformation moléculaire de l'acide aminé (AA) telle qu'elle dévie à droite le plan de polarisation de la lumière (conformation dextrogyre ou D-aa), et une conformation moléculaire de l'acide aminé (aa) telle qu'elle dévie à gauche le plan de polarisation de la lumière (conformation lévogyre ou L-aa). La nature n'a retenu pour les acides aminés naturels que la conformation lévogyre. En conséquence, un peptide d'origine naturelle ne sera constitué que d'acides aminés de type L-aa. Cependant, la synthèse chimique en laboratoire permet de préparer des acides aminés ayant les deux conformations possibles. A partir de ce matériel de base, il est ainsi possible d'incorporer lors de la synthèse de peptide aussi bien des acides aminés sous forme d'isomères optiques dextrogyre ou lévogyre. Ainsi, les acides aminés constituant le peptide peuvent peuvent être sous configuration L- et D- ; de manière préférentielle, les acides aminés sont sous forme L. Le peptide de formule générale (I) peut donc être sous forme L-, D-ou DL-.

Le peptide de formule générale (I) peut être obtenu soit par synthèse chimique classique (en phase solide ou en phase homogène liquide), soit par synthèse enzymatique (Kullman et al., J. Biol. Chem. 1980, 225, 8234), à partir d'acides aminés constitutifs ou de leurs dérivés.

Le peptide de formule générale (1) peut également être obtenu par fermentation d'une souche de bactéries modifiées ou non, par génie génétique, ou encore par extraction de protéines d'origine animale ou végétale, préférentiellement d'origine végétale, suivie d'une hydrolyse contrôlée qui libère des fragments peptidiques correspondant totalement ou partiellement aux peptides de formule générale (I).

De très nombreuses protéines trouvées dans les plantes sont susceptibles de contenir ces séquences au sein de leur structure. L'hydrolyse ménagée permet de dégager ces fragments peptidiques. Il est possible, mais non nécessaire pour réaliser l'invention, d'extraire soit les protéines concernées d'abord et de les hydrolyser ensuite, soit d'effectuer l'hydrolyse d'abord sur un extrait brut et de purifier les fragments peptidiques ensuite. Il est également possible d'utiliser certains extraits hydrolysés sans en purifier les fragments peptidiques correspondant aux peptides de formule générale (I) selon l'invention, mais en s'assurant toutefois de la présence desdits fragments par des moyens analytiques appropriés.

D'autres procédés plus simples ou plus complexes peuvent être envisagés par l'homme du métier connaissant le métier de synthèse, d'extraction et de purification des protéines et des peptides. Ainsi le peptide de formule générale (I) peut être d'origine naturelle ou synthétique. Préférentiellement, le peptide est obtenu par synthèse chimique.

Le principe actif peut être un mélange de dérivés peptidiques et/ou constitués de dérivés d'acides aminés.

Selon un mode de réalisation avantageux, le principe actif est préalablement solubilisé dans un ou plusieurs solvants classiquement utilisés par l'homme du métier, comme l'eau, le glycérol, l'éthanol, le propylène glycol, le butylène glycol, le dipropylène glycol, les diglycols éthoxylés ou propoxylés, les polyols cycliques, la vaseline, une huile végétale ou tout mélange de ces solvants.

Selon encore un autre mode de réalisation avantageux, le principe actif est préalablement solubilisé dans un vecteur cosmétique ou pharmaceutique comme les liposomes ou adsorbés sur des polymères organiques poudreux, des supports minéraux comme les talcs et bentonites, et plus généralement solubilisés dans, ou fixés sur, tout vecteur cosmétiquement ou pharmaceutiquement acceptable.

Il est bien entendu que le principe actif peut être utilisé seul ou bien en association avec au moins un autre principe actif, dans une composition cosmétique ou pour la préparation d'une composition pharmaceutique et/ou dermatologique.
Les compositions selon l'invention pourront être appliquées par toute voie appropriée, notamment orale, parentérale ou topique externe, et leur formulation sera adaptée par l'homme du métier, en particulier pour des compositions cosmétiques ou dermatologiques. Avantageusement, les compositions selon l'invention se présentent sous une forme adaptée à l'application par voie topique. Ces compositions doivent donc contenir un milieu cosmétiquement et/ou dermatologiquement acceptable, c'est-à-dire compatible avec la peau et les phanères, et couvrent toutes les formes cosmétiques ou dermatologiques. Ces compositions pourront notamment être sous forme de crèmes, émulsions huile-dans-eau, ou eau-dans-huile ou émulsions multiples, solutions, suspensions, gels, laits, lotions, sticks ou encore de poudres, adaptés à une application sur la peau, les lèvres et/ou les phanères.

Ces compositions comprennent les excipients nécessaires à leur formulation, tels que solvants, épaississants, diluants, tensioactifs, anti-oxydants, colorants, conservateurs, parfums.

Bien entendu, l'homme de métier veillera à choisir les éventuels composés complémentaires, actifs ou non-actifs, et/ou leur quantité, de telle sorte que les propriétés avantageuses du mélange ne soient pas, ou sensiblement pas, altérées par l'adjonction envisagée.

La composition utilisable selon l'invention peut en particulier consister en une composition pour soins capillaires, et notamment un shampooing, un après-shampooing, une lotion de mise en plis, une lotion traitante, une crème ou un gel coiffant, une lotion restructurante pour les cheveux, un masque, etc. La composition cosmétique selon l'invention peut être utilisée notamment dans les traitements mettant en oeuvre une application qui est suivie ou non suivie d'un rinçage, ou encore sous forme de shampooing.

Elle peut également se présenter sous forme de teinture ou de mascara à appliquer au pinceau ou au peigne, en particulier sur les cils, les sourcils ou les cheveux.

Avantageusement, les compositions utilisables contiennent en outre au moins un autre agent actif favorisant l'action des peptides de formule générale (I). Ainsi, la composition peut associer, au principe actif, des agents actifs ayant une action antioxydante, ou encore stimulant la synthèse des macromolécules dermiques, ou encore stimulant le métabolisme énergétique. Par exemple, en tant qu'agents actifs ayant une action antioxydante, on peut citer la vitamine C, la vitamine E, ou les extraits polyphénoliques de plantes.

On peut également citer, en tant qu'agents actifs stimulant les synthèses des macromolécules dermiques (laminine, fibronectine, collagène), par exemple le peptide de collagène commercialisé sous le nom « Collaxyl®» par la société Vincience.
Enfin, en tant qu'agents actifs stimulant le métabolisme énergétique, on peut citer le principe actif commercialisé sous la dénomination « GP4G® » par la société Vincience.
Selon un autre aspect, la composition selon l'invention peut être une composition solaire, c'est-à-dire une composition aidant à la protection contre le rayonnement solaire. Ainsi, il peut être avantageusement ajouté, à la composition selon l'invention, des actifs aidant à la protection solaire tels que, par exemple, des filtres solaires.
Il est bien évident que l'invention s'adresse aux mammifères en général, et plus particulièrement aux êtres humains.

La quantité efficace de principe actif correspond à la quantité de peptide de formule générale (I) nécessaire pour obtenir le résultat recherché, à savoir : augmenter la synthèse d'ATP, protéger la peau des dommages oxydatifs et plus généralement, protéger la peau des agressions extérieures et prévenir ou traiter le vieillissement cutané.

Selon un mode de réalisation avantageux, le principe actif de formule générale (I) est présent dans les compositions de l'invention à une concentration comprise entre 0,0005 et 500 ppm (parties par million) environ, et préférentiellement à une concentration comprise entre 0,01 et 5 ppm environ par rapport au poids total de la composition finale.

Ces compositions pourront notamment se présenter sous forme d'une solution aqueuse, hydroalcoolique ou huileuse ; d'une émulsion huile-dans-eau, eau-dans-huile ou émulsions multiples ; elles peuvent aussi se présenter sous forme de crèmes, de suspensions, ou encore de poudres, adaptées à une application sur la peau, les muqueuses, les lèvres et/ou les phanères. Ces compositions peuvent être plus ou moins fluides et avoir l'aspect d'une crème, d'une lotion, d'un lait, d'un sérum, d'une pommade, d'un gel, d'une pâte ou d'une mousse. Elles peuvent aussi se présenter sous forme solide, comme un stick ou être appliquées sur la peau sous forme d'aérosol. Elles peuvent être utilisées comme produit de soin et/ou comme produit de maquillage de la peau.

Ces compositions comprennent, en outre, tout additif communément utilisé dans le domaine d'application envisagé ainsi que les adjuvants nécessaires à leur formulation, tels que des solvants, des épaississants, des diluants, des anti-oxydants, des colorants, des filtres solaires, des principes auto-bronzants, des pigments, des charges, des conservateurs, des parfums, des absorbeurs d'odeur, des actifs cosmétiques ou pharmaceutiques, des huiles essentielles, des vitamines, des acides gras essentiels, des tensioactifs, des polymères filmogènes, etc.

Dans tous les cas, l'homme du métier veillera à ce que ces adjuvants ainsi que leurs proportions soient choisis de telle manière à ne pas nuire aux propriétés avantageuses recherchées de la composition selon l'invention. Ces adjuvants peuvent, par exemple, correspondre à 0,01 à 20 % du poids total de la composition. Lorsque la composition de l'invention est une émulsion, la phase grasse peut représenter de 5 à 80 % en poids et de préférence de 5 à 50 % en poids par rapport au poids total de la composition. Les émulsionnants et co-émulsionnants utilisés dans la composition seront choisis parmi ceux classiquement utilisés dans le domaine considéré. Par exemple, ils peuvent être utilisés en une proportion allant de 0,3 à 30 % en poids, par rapport au poids total de la composition.

Par ses activités particulières, le principe actif pourra être utilisé avantageusement dans une composition cosmétique ou pour la préparation d'une composition pharmaceutique.

En particulier, le principe actif pourra être utilisé avantageusement dans une composition cosmétique pour augmenter la synthèse d'ATP intracellulaire des cellules de la peau.

Le principe actif pourra également être utilisé avantageusement dans une composition cosmétique pour augmenter l'activité ou la synthèse de l'enzyme transprényl transférase et/ou du coenzyme Q10 dans les cellules de la peau.

L'utilisation du principe actif selon l'invention, dans une composition cosmétique pour protéger la peau et les phanères des dommages oxydatifs est décrite. Ledit principe actif est avantageusement utilisé en tant que principe actif antioxydant, et/ou en tant que principe actif anti-radicalaire, et/ou en tant que principe actif anti-glycation. Par principe actif anti-radicalaire, on entend tout composé capable de piéger les radicaux libres avant les étapes ultimes de dégradation des constituants biologiques de la peau, on parle alors de composés antioxydants. Par principe actif anti-glycation, on entend tout composé capable de limiter les dommages cellulaires causés par les réactions de glycation ou de glycoxydation. Ainsi, le principe actif permettra de lutter contre les dommages esthétiques provoqués sur la peau et/ou les cheveux par les radicaux libres.

Aussi, l'agent actif peut être utilisé avantageusement dans une composition cosmétique pour la protection de la peau et des phanères contre tous les types d'agressions extérieures. On entend, par l'expression « agression extérieure », les agressions que peut produire l'environnement. A titre d'exemple, on peut citer des agressions telles que la pollution, les rayonnements UV, ou encore les produits à caractère irritant tels que les tensioactifs, les conservateurs ou les parfums. Par pollution, on entend aussi bien la pollution « extérieure », due par exemple aux particules de diesel, à l'ozone ou aux métaux lourds, que la pollution « intérieure » qui peut être due notamment aux émissions de solvants de peintures, de colles, ou de papier-peints (tels que toluène, styrène, xylène ou benzaldehyde), ou bien encore la fumée de cigarette.

L'utilisation dudit principe actif dans une composition cosmétique ou pour la préparation d'une composition pharmaceutique, en tant que principe actif photo-protecteur et, plus particulièrement, en tant que principe actif photo-protecteur dit « secondaire » est généralement décrite. On distingue, en effet, les principes actifs photo-protecteurs primaires des principes actifs photo-protecteurs secondaires. Les principes actifs photo-protecteurs primaires sont des substances qui exercent un pouvoir physique : ils sont en mesure d'absorber les rayonnements UV et de les restituer sous forme de chaleur afin de protéger la peau. Les principes actifs photo-protecteurs secondaires sont des substances qui ont plutôt un effet biologique ; ce sont, par exemple, les principes actifs de type antioxydant qui interrompent les chaînes de réactions photochimiques qui sont déclenchées lorsque le rayonnement UV pénètre dans la peau.

Dans un autre aspect essentiel, le principe actif pourra être utilisé avantageusement dans une composition cosmétique pour lutter de manière préventive et/ou curative contre les signes du vieillissement cutané, et plus spécifiquement, pour lutter contre et/ou prévenir le vieillissement photo-induit (photo-vieillissement). Par signes cutanées du vieillissement, on entend toutes modifications de l'aspect extérieur de la peau et des phanères dues au vieillissement comme, par exemple, les rides et ridules, la peau flétrie, la peau molle, la peau amincie, le manque d'élasticité et/ou de tonus de la peau, la peau terne et sans éclat ou les taches de pigmentation de la peau, la décoloration des cheveux ou les taches sur les ongles, mais également toute modification interne de la peau qui ne se traduit pas systématiquement par un aspect extérieur modifié comme, par exemple, tout dommage oxydatif de la peau consécutif à une exposition aux rayonnements ultraviolets (UV). Le principe actif, ou la composition le contenant, permettra de lutter, en particulier, contre la perte d'élasticité et de fermeté de la peau.

L'utilisation dans une composition cosmétique d'une quantité efficace de principe actif tel que décrit précédemment, pour prévenir les dommages causés à la peau par une exposition au soleil ou une exposition à des rayonnements ionisants lors de radiothérapies est également décrite.

L'utilisation dans une composition cosmétique, d'une quantité efficace de principe actif tel que décrit précédemment, pour stimuler et/ou protéger les mitochondries, en particulier sur les zones du corps exposées aux rayonnements UV est généralement décrite.

Une composition pharmaceutique caractérisée en ce que le principe actif selon l'invention est formulé pour atténuer une pathologie liée à des processus d'oxydation, ou encore certaines pathologies du vieillissement est également décrite.

D'autres avantages et caractéristiques de l'invention apparaîtront mieux à la lecture des exemples donnés à titre illustratif et non limitatif.

### Exemple 1 : Mise en évidence de l'effet activateur du peptide SEQ ID n°2 sur la synthèse d'ATP intracellulaire

Le but de cette étude est de déterminer l'influence du peptide SEQ ID n°2 sur la synthèse d'ATP.

### Protocole

Cette étude a été effectuée à l'aide d'un Kit « ATP Bioluminescence Assay Kit HS II » (Roche Applied Science) :

Les fibroblastes dermiques sont traités avec une solution à 1 %, d'une solution à 50 ppm, contenant le peptide SEQ ID n°2, représentatif de la famille de peptides selon l'invention, pendant une période allant de 1 à 3 heures. A la fin des temps d'incubation, les puits sont vidés de leur milieu et rincés avec 2 ml de PBS froid avant d'ajouter 250 µl d'un tampon de lyse fourni par le kit. Les cellules de chaque puits sont ensuite grattées puis récoltées dans des tubes de 14 ml. Chaque puits est rincé avec 2 x 500 µl de PBS froid et le tout est de nouveau récolté dans les tubes respectifs. A partir de ces échantillons, une dilution est réalisée au 1/12000^{ième} dans du PBS froid avant chaque lecture. Le dosage d'ATP est réalisé sur ces échantillons : 50 µL de cette dilution sont déposés dans une luma-cuvette et 50 µL de luminol sont ajoutés. Après 10 secondes, la lecture de la luminescence est déclenchée. Les valeurs sont standardisées par rapport à la quantité de protéines pour chaque échantillon. Les mesures sont effectuées à l'aide d'un appareil : le Biocounter M2010A LUMAC®/3M.

### Résultats

Les dosages d'ATP montrent qu'il y a une augmentation de la quantité d'ATP intracellulaire de 17 % après 1 heure et de 67% après 3 heures de culture dans des cellules traitées par le peptide SEQ ID N°2, en comparaison des cellules non traitées.

### Conclusion

Le peptide SEQ ID n°2 active fortement la synthèse d'ATP intracellulaire dans les cellules cutanées.

### Exemple 2 : Evaluation de l'effet protecteur du peptide SEQ ID n°1 vis-à-vis des dommages oxydatifs

Le but de cette étude est de déterminer l'effet protecteur du peptide SEQ ID n° 1 vis-à-vis de fibroblastes dermiques soumis à un stress oxydatif provoqué par des rayonnements UVB ou par de l'eau oxygénée (H₂O₂). Pour évaluer les dommages oxydatifs subis par les cellules, des dosages de la carbonylation des protéines ont été réalisés.

La carbonylation des protéines résulte du clivage oxydatif des protéines ou d'une oxydation des résidus arginine, lysine, proline ou thréonine. Le dosage de la carbonylation des protéines s'effectue par une technique EIA (Enzyme Immuno Assay).

### Protocole

Des fibroblastes en culture ont été mis en présence du peptide SEQ ID n° 1 à 1 %, d'une solution à 50 ppm, 72 heures avant, pendant, et encore 24 heures après le stress oxydatif (irradiation aux UVB à 50 mJ/cm² ou traitement par 2 mM d'H₂O₂). Des contrôles non traités et non soumis au stress oxydatif sont réalisés.

La mesure de la carbonylation consiste à utiliser du DNP (dinitrophényl) qui a la propriété de se fixer spécifiquement sur les groupements carbonyles des protéines. Le DNP fixé sera ensuite dosé par une méthode ELISA, grâce à un anticorps anti-DNP couplé à une peroxydase. Une gamme de BSA (bovine sérum albumine) oxydée (dont on connaît la concentration en groupements carbonyles) est utilisée pour l'étalonnage.

### Résultats

Les résultats obtenus montrent une diminution de 30% de la carbonylation des protéines lorsque les cellules sont traitées avec le peptide SEQ ID n°1 selon l'invention, en comparaison avec les cellules non traitées.

On observe plus particulièrement une diminution de 20 % de la carbonylation lorsque les cellules traitées avec le peptide SEQ ID n°1 sont soumises à une irradiation par les UVB ou à un stress oxydatif par l'H₂O₂, par comparaison aux cellules irradiées ou stressées mais non traitées avec le principe actif.

### Conclusion

Le peptide SEQ ID n°1 protège efficacement les cellules cutanées contre les dommages oxydatifs provoqués par des rayonnements UVB ou de l'eau oxygénée.

### Exemple 3 : Evaluation de l'effet protecteur du peptide SEQ ID n°1 vis-à-vis d'un stress induit par la glycation

Le but de cette étude est de déterminer l'effet protecteur du peptide SEQ ID n°1, vis-à-vis de culture d'épiderme *ex vivo* soumises à un stress par un agent glycant.

### Protocole

Des biopsies de peau humaine sont maintenues en culture *ex vivo*, traitées avec une solution à 1 %, d'une solution mère à 50 ppm de peptide SEQ ID n°1, 24 heures avant, et encore 24 heures après la mise en présence avec un agent glycant (méthyl glyoxal à 5 ou 10 mM). Des coupes et des colorations histologiques hématoxyline-éosine (H&E) permettent d'évaluer la qualité des structures cutanées.

### Résultats

L'évaluation des structures cutanées montrent une nette résistance au stress cellulaire induit par la glycation des biopsies de peau traitées par le peptide SEQ ID n°1.

### Conclusion

Le peptide SEQ ID n° 1 protège la peau d'un stress induit par la glycation.

### Exemple 4 : Préparation de compositions

### 1 - Crème protection solaire:

| Noms commerciaux | Noms INCI | % massique |
|---|---|---|
| PHASE A | | |
| Eau déminéralisée | Aqua (Water) | qsp |
| Pemulen TR1 | Acrylates/C10-30 Alkyl Acrylate Crosspolymer | 0,40 |
| Glycerine | Glycerin | 3,00 |
| Nipastat Sodium | Sodium Methylparaben (and) Sodium Ethylparaben (and) Sodium Butyl paraben (and) Sodium Propylparaben (and) Sodium Isobutylparaben | 0,15 |

| PHASE B | | |
|---|---|---|
| Parsol MCX | Ethylhexyl Methoxycinnamate | 7,50 |
| Eusolex 4360 | Benzophenone-3 | 3,00 |
| Parsol 1789 | Butyl Methoxydibenzoylmethane | 2,00 |
| Myritol 318 | Caprylic/Capric Triglyceride | 4,00 |
| Emulgade SEV | Hydrogenated Palm Glycerides (and) Ceteareth-20 (and) Ceteareth-12 (and) Cetearyl Alcohol | 5,00 |
| Propylparaben | Propylparaben | 0,15 |
| Nacol 16-98 | Cetyl Alcohol | 1,00 |

| PHASE C | | |
|---|---|---|
| TEA | Triethanolamine | 0,20 |

| PHASE D | | |
|---|---|---|
| Peptide SEQ ID n° 1 | | 3 ppm |
| Parfum | Parfum (Fragrance) | qsp |
| Colorant | | qsp |

Les constituants de la phase A et de la phase B sont chauffés séparément entre 70°C et 75°C. La phase B est émulsionnée dans la phase A sous agitation. La phase C est ajoutée, à 45°C, en augmentant l'agitation. La phase D est ensuite additionnée lorsque la température se situe en dessous de 40°C. Le refroidissement est poursuivi jusqu'à 25°C sous vive agitation.

### 2 -Lait auprès-soleil :

| Noms commerciaux | Noms INCI | % massique |
|---|---|---|
| PHASE A | | |
| Montanov L | C14-22 Alcohols (and) C12-20 Alkyl Glucoside | 3,00 |
| Waglinol 2559 | Cetearyl Isononanoate | 4,00 |
| Tegosoft TN | C12-15 Alkyl Benzoate | 3,00 |
| Huile de Noyaux d'Abricot | Prunus Armeniaca (Apricot) Kernel Oil | 2,00 |
| Huile d'Avocat | Persea Gratissima (Avocado) Oil | 1,00 |
| Abil 350 | Dimethicone | 1,00 |

| PHASE B | | |
|---|---|---|
| Eau déminéralisée | Aqua (Water) | qsp |

| PHASE C | | |
|---|---|---|
| Simulgel EG | Sodium Acrylate/Acryloyldimethyl Taurate | 0,4 |
| | Copolymer (and) Isohexadecane (and) Polysorbate 80 | |
| | Copolymer (and) Polysorbate 80 | |

| PHASE D | | |
|---|---|---|
| Phenonip | Phenoxyethanol (and) Methylparaben (and) | 0,30 |
| | Ethylparaben (and) Butylparaben (and) Propylparaben (and) Isobutylparaben | |
| | Ethylparaben and | |
| | Propylparaben and | |

| Noms commerciaux | Noms INCI | % massique |
|---|---|---|
| | Buthylparaben | |
| Germall 115 | Imidazolidinyl Urea | 0,20 |

| PHASE E | | |
|---|---|---|
| Peptide SEQ ID n° 2 | | 0,1 ppm |

Préparer la phase A sous agitation. Incorporer la gomme xanthane progressivement, sous agitation défloculeuse. Les phases C et D seront incorporées une fois le gel terminé. La phase E, préparée préalablement jusqu'à parfaite dissolution de la DHA, sera rajoutée ensuite. Ajuster le pH si nécessaire à 4 - 4,5. Colorer et parfumer.

### 3 -Crème anti-äge :

| ***Noms commerciaux*** | ***Noms INCI*** | **% *massique*** |
|---|---|---|
| ***Phase A*** | | |
| Montanov 68 | Cetearyl Alcohol (and) Cetearyl Glucoside | 6,00 |
| Squalane | Squalane | 3,00 |
| Cetiol SB 45 | Butyrospermum Parkii ( Shea Butter) | 2,00 |
| Waglinol 250 | Cetearyl Ethylhexanoate | 3,00 |
| Amerchol L- 101 | Mineral Oil (and) Lanolin Alcohol | 2,00 |
| Abil 350 | Dimethicone | 1,50 |
| BHT | BHT | 0,01 |

| ***Phase B*** | | |
|---|---|---|
| Huile d'Avocat | Persea Gratissima (Avocado) Oil | 1,25 |
| Phenonip | Phenoxyethartol (and) Methylparaben (and) Ethylparaben (and) Butylparaben (and) Propylparaben (and) Isobutylparaben | 0,75 |

| ***Phase C*** | | |
|---|---|---|
| ***Noms commerciaux*** | ***Noms INCI*** | ***% massique*** |
| Eau déminéralisée | Aqua (Water) | qsp |
| Butylene Glycol | Butylene Glycol | 2,00 |
| Glucam E10 | Methyl Gluceth-10 | 1.00 |
| Allantoin | Allantoin | 0,15 |
| Carbopol Ultrez 10 | Carbomer | 0,20 |

| ***Phase D*** | | |
|---|---|---|
| TEA | Triethanolamine | 0,18 |

| ***Phase E*** | | |
|---|---|---|
| Peptide SEQ ID n° 1 | | 0,5 ppm |
| GP4G | Water (and) Artemia Extract | 1,50 |
| Collaxyl | Water (and) Butylene Glycol (and) Hexapeptide-9 | 3,00 |

| ***Phase F*** | | |
|---|---|---|
| Parfum | Parfum (Fragrance) | qsp |
| Colorant | | qsp |

Préparer et fondre la phase A à 65-70°C. Chauffer la phase C à 65-70°C. La phase B est ajoutée à la phase A juste avant d'émulsionner A dans B. A environ 45°C, le carbomer est neutralisé par addition de la phase D. La phase E est ensuite additionnée sous légère agitation et le refroidissement est poursuivi jusqu'à 25°C. La phase F est alors additionnée si souhaité.

### 4 -Crème protectrice de jour :

| ***Noms commerciaux*** | ***Noms INCI*** | ***% massique*** |
|---|---|---|
| ***Phase A*** | | |
| Emulium Delta | Cetyl alcohol (and) Glyceryl Stearate (and) PEG-75 Stearate (and) Ceteth-20 (and) Steareth-20 | 4,00 |
| Lanette O | Cetearyl Alcohol | 1,50 |
| D C 200 Fluid/100cs | Dimethicone | 1,00 |
| DUB 810C | Coco Caprylate/Caprate | 1,00 |
| DPPG | Propylene Glycol Dipelargonate | 3,00 |
| DUB DPHCC | Dipentaerythrityl Hexacaprylate/Hexacaprate | 1,50 |
| Cegesoft PS6 | Vegetable Oil | 1,00 |
| Vitamine E | Tocopherol | 0,30 |
| Phenonip | Phenoxyethanol (and) Methylparaben (and) Ethylparaben (and) Butylparaben (and) Propylparaben (and) Isobutylparaben | 0,70 |

| ***Phase B*** | | |
|---|---|---|
| Eau déminéralisée | Aqua | qsp 100 |
| Glycerine | Glycerin | 2,00 |
| Carbopol EDT 2020 | Acrylates/C 10-30A1ky1 Acrylate Crosspolymer | 0,15 |
| Keltrol BT | Xanthan Gum | 0,30 |

| ***Phase C*** | | |
|---|---|---|
| Sodium Hydroxide (sol.à 10%) | Sodium Hydroxide | 0,30 |

| ***Phase D*** | | |
|---|---|---|
| Eau déminéralisée | Aqua | 5,00 |
| Stay-C 50 | Sodium Ascorbyl Phosphate | 0,50 |

| ***Phase E*** | | |
|---|---|---|
| Butylene Glycol | Butylene Glycol | 2,00 |
| Dekaben CP | Chlorphenesin | 0,20 |

| ***Phase F*** | | |
|---|---|---|
| GP4G | Water (and) Artemia Extract | 1,00 |
| Peptide SEQ ID n°2 | | 5 ppm |

Préparer la phase A et chauffer à 75°C sous agitation. Préparer la phase B en dispersant le carbopol, puis la gomme xanthane sous agitation. Laisser reposer. Chauffer à 75°C.
A température, émulsionner A dans B sous agitation rotor-stator. Neutraliser avec la phase C sous agitation rapide. Après refroidissement à 40°C, additionner la phase D, puis la phase E. Le refroidissement est poursuivi sous agitation légère et la phase F rajoutée.

## Revendications

1. Composition **caractérisée en ce qu'**elle contient en tant que principe actif, dans un milieu physiologiquement acceptable, au moins un peptide comprenant de 5 à 13 résidus d'acides aminés et dont la séquence répond à la formule générale (I) :
R₁-(AA)ₙ- Ala-Val- Leu- Ala-Gly-(AA)ₚ-R₂
dans laquelle
AA représente un acide aminé quelconque, ou un de ses dérivés, et n et p sont des nombres entiers compris entre 0 et 4,
R₁ représente la fonction amine primaire de l'acide aminé N-terminal ou un groupement protecteur pouvant être choisi parmi un groupement acétyle, un groupement benzoyle, un groupement tosyle ou un groupement benzyloxycarbonyle,
R₂ représente la fonction hydroxyle de l'acide carboxyle de l'acide aminé C-terminal, libre ou substituée par un groupement protecteur pouvant être choisi parmi une chaîne alkyle de C₁ à C₂₀, ou un groupement NH₂, NHY ou NYY avec Y représentant une chaîne alkyle de C₁ à C₄.

2. Composition selon la revendication 1, **caractérisée en ce que** le peptide de formule générale (I) correspond à la séquence SEQ ID n°1 Ala-Val-Leu-Ala-Gly-Asp-NH2.

3. Composition selon la revendication 1 **caractérisée en ce que** le peptide de formule générale (I) correspond à la séquence SEQ ID n°2 Ala-Val-Leu-Ala-Gly-Asp.

4. Composition selon la revendication 1 **caractérisée en ce que** le peptide de formule générale (I) correspond à la séquence SEQ ID Ala-Val-Leu-Ala-Gly-NH2.

5. Composition selon la revendication 1 **caractérisée en ce que** le peptide de formule générale (I) correspond à la séquence SEQ ID Ala-Val-Leu-Ala-Gly.

6. Composition selon l'une des revendications 1 à 5, **caractérisée en ce que** le peptide de formule générale (I) possède au moins un groupement fonctionnel protégé par un groupement protecteur, ce groupement protecteur étant soit une acylation ou une acétylation de l'extrémité amino-terminale, soit une amidation ou une estérification de l'extrémité carboxy-terminale, soit les deux.

7. Composition selon l'une quelconque des revendications précédentes, **caractérisée en ce que** le principe actif est présent dans la composition à une concentration comprise entre 0,0005 et 500 ppm environ, et préférentiellement à une concentration comprise entre 0,01 et 5 ppm par rapport au poids total de la composition.

8. Composition selon l'une quelconque des revendications précédentes, **caractérisée en ce que** le principe actif est préalablement solubilisé dans un ou plusieurs solvants cosmétiquement ou pharmaceutiquement acceptables, comme l'eau, le glycérol, l'éthanol, le propylène glycol, le butylène glycol, le dipropylène glycol, les diglycols éthoxylés ou propoxylés, les polyols cycliques, la vaseline, une huile végétale ou tout mélange de ces solvants.

9. Composition selon l'une quelconque des revendications précédentes, **caractérisée en ce qu'**elle se présente sous une forme adaptée à l'application par voie topique.

10. Composition selon l'une quelconque des revendications précédentes, **caractérisée en ce qu'**elle contient, en outre, au moins un autre agent actif choisi parmi la vitamine C, la vitamine E, les extraits polyphénoliques de plantes, le peptide de collagène commercialisé sous le nom de Collaxyl®, et le principe actif commercialisé sous la dénomination GP4G®.

11. Utilisation cosmétique d'une composition comprenant un principe actif tel que défini dans l'une quelconque des revendications 1 à 6.

12. Composition comprenant un principe actif tel que défini dans l'une quelconque des revendications 1 à 6, pour son utilisation dans un traitement dermatologique.

13. Composition comprenant un principe actif tel que défini dans l'une quelconque des revendications 1 à 6 pour augmenter la synthèse d'ATP intracellulaire des cellules de la peau.

14. Composition comprenant un principe actif tel que défini dans l'une quelconque des revendications 1 à 6 pour augmenter l'activité ou la synthèse de l'enzyme transprényl transférase et/ou de coenzyme Q10 dans les cellules de la peau.

15. Composition comprenant un principe actif tel que défini dans l'une quelconque des revendications 1 à 6 pour protéger la peau et les phanères contre tous les types d'agressions extérieures.

16. Composition selon la revendication 15 pour prévenir ou traiter les dommages causés à la peau et aux phanères par les rayonnements UV.

17. Composition selon la revendication 15 pour protéger la peau et les phanères des dommages oxydatifs.

18. Utilisation cosmétique selon la revendication 11 pour prévenir ou traiter les signes cutanés du vieillissement et/ou du photo-vieillissement.

## Patentansprüche

1. Zusammensetzung, **dadurch gekennzeichnet, dass** sie als Wirkstoff, in einem physiologisch akzeptablen Medium, mindestens ein Peptid enthält, umfassend von 5 bis 13 Aminosäurereste, und wovon die Sequenz der allgemeinen Formel (I) entspricht:
R₁-(AA)ₙ- Ala-Val- Leu- Ala-Gly-(AA)ₚ-R₂
wobei
AA irgendeine Aminosäure darstellt oder eines ihrer Derivate, und n und p ganze Zahlen zwischen 0 und 4 sind,
R₁ die primäre Aminofunktion der N-terminalen Aminosäure oder eine Schutzgruppe darstellt, die aus einer Acetylgruppe, einer Benzoylgruppe, einer Tosylgruppe oder einer Benzyloxycarbonylgruppe gewählt werden kann,
R₂ die Hydroxylfunktion der Carboxylsäure der C-terminalen Aminosäure darstellt,
frei oder substituiert durch eine Schutzgruppe, die aus einer Alkylkette von C₁ bis C₂₀ oder einer NH₂-, NHY- oder NYY-Gruppe gewählt werden kann, wobei Y eine Alkylkette von C₁ bis C₄ darstellt.

2. Zusammensetzung nach Anspruch 1, **dadurch gekennzeichnet, dass** das Peptid mit der allgemeinen Formel (I) der Sequenz SEQ ID Nr. 1 Ala-Val-Leu-Ala-Gly-Asp-NH2 entspricht.

3. Zusammensetzung nach Anspruch 1, **dadurch gekennzeichnet, dass** das Peptid mit der allgemeinen Formel (I) der Sequenz SEQ ID Nr. 2 Ala-Val-Leu-Ala-Gly-Asp entspricht.

4. Zusammensetzung nach Anspruch 1, **dadurch gekennzeichnet, dass** das Peptid mit der allgemeinen Formel (I) der Sequenz SEQ ID Ala-Val-Leu-Ala-Gly-NH2 entspricht.

5. Zusammensetzung nach Anspruch 1, **dadurch gekennzeichnet, dass** das Peptid mit der allgemeinen Formel (I) der Sequenz SEQ ID Ala-Val-Leu-Ala-Gly entspricht.

6. Zusammensetzung nach einem der Ansprüche 1 bis 5, **dadurch gekennzeichnet, dass** das Peptid mit der allgemeinen Formel (I) mindestens eine funktionelle Gruppe aufweist, die von einer Schutzgruppe geschützt wird, wobei diese Schutzgruppe entweder eine Acylierung oder eine Acetylierung des amino-terminalen Endes ist,oder eine Amidbildung oder eine Veresterung des carboxy-terminalen Endes oder beides ist.

7. Zusammensetzung nach einem der vorhergehenden Ansprüche, **dadurch gekennzeichnet, dass** der Wirkstoff in der Zusammensetzung in einer Konzentration zwischen ungefähr 0,0005 und 500 ppm und vorzugsweise in einer Konzentration zwischen 0,01 und 5 ppm mit Bezug auf das Gesamtgewicht der Zusammensetzung vorhanden ist.

8. Zusammensetzung nach einem der vorhergehenden Ansprüche, **dadurch gekennzeichnet, dass** der Wirkstoff zuvor in einem oder in mehreren kosmetisch oder pharmazeutisch akzeptablen Lösemitteln wie z.B. Wasser, Glycerol, Ethanol, Propylenglycol, Butylenglycol, Dipropylinglycol, ethoxyliertne oder propoxylierten Diglykolen, cyclischen Polyolen, Vaselin, einem pflanzlichen Öl oder jeder Mischung dieser Lösemittel solubilisiert wird.

9. Zusammensetzung nach einem der vorhergehenden Ansprüche, **dadurch gekennzeichnet, dass** sie eine Form aufweist, die an die Anwendung auf topischem Weg angepasst ist.

10. Zusammensetzung nach einem der vorhergehenden Ansprüche, **dadurch gekennzeichnet, dass** sie außerdem mindestens einen weiteren Wirkstoff umfasst, ausgewählt aus Vitamin C, Vitamin E, den Polyphenolextrakten von Pflanzen, dem Collagenpeptid, das unter dem Namen Collaxyl® vertrieben wird, und dem Wirkstoff, der unter der Bezeichnung GP4G® vertrieben wird.

11. Kosmetische Verwendung einer Zusammensetzung, umfassend einen Wirkstoff wie in einem der Ansprüche 1 bis 6 definiert.

12. Zusammensetzung, umfassend einen Wirkstoff wie in einem der Ansprüche 1 bis 6 definiert, für seine Verwendung bei einer dermatologischen Behandlung.

13. Zusammensetzung, umfassend einen Wirkstoff wie in einem der Ansprüche 1 bis 6 definiert, um die Synthese des intrazellulären ATP der Zellen der Haut zu erhöhen.

14. Zusammensetzung, umfassend einen Wirkstoff wie in einem der Ansprüche 1 bis 6 definiert, um die Aktivität oder die Synthese des Enzyms Transprenyltransferase und/oder des Coenzyms Q10 in den Zellen der Haut zu erhöhen.

15. Zusammensetzung, umfassend einen Wirkstoff wie in einem der Ansprüche 1 bis 6 definiert, um die Haut und die Hautanhangsgebilde gegen alle Arten von äußeren Aggressionen zu schützen.

16. Zusammensetzung nach Anspruch 15, um den Schäden vorzubeugen oder diese zu behandeln, die an der Haut und an den Hautanhangsgebilden durch die UV-Strahlungen verursacht werden.

17. Zusammensetzung nach Anspruch 15, um die Haut und die Hautanhangsgebilde vor oxidativen Schäden zu schützen.

18. Kosmetische Verwendung nach Anspruch 11, um den Anzeichen der Haut der Alterung und/oder der Lichtalterung vorzubeugen und/oder diese zu behandeln.

## Claims

1. Composition **characterised in that** it contains, as an active ingredient, in a physiologically acceptable medium, at least one peptide comprising 5 to 13 amino acid residues and wherein the sequence complies with general formula (I):
R₁- (AA)ₙ-Ala-Val-Leu-Ala-Gly- (AA)ₚ-R₂
wherein
AA is any amino acid, or any derivative thereof, and n and p are integers between 0 and 4,
R₁ is the primary amine function of the N-terminal amino acid or a protecting group suitable for being chosen from an acetyl group, a benzoyl group, a tosyl group or a benzyloxycarbonyl group,
R₂ is the hydroxyl function of the carboxyl acid of the C-terminal amino acid, free or substituted by a protecting group suitable for being chosen from a C₁ to C₂₀ alkyl chain, or an NH₂, NHY or NYY group where Y is a C₁ to C₄ alkyl chain.

2. Composition according to claim 1, **characterised in that** the peptide according to general formula (I) corresponds to the sequence SEQ ID No. 1 Ala-Val-Leu-Ala-Gly-Asp-NH2.

3. Composition according to claim 1, **characterised in that** the peptide according to general formula (I) corresponds to the sequence SEQ ID No. 2 Ala-Val-Leu-Ala-Gly-Asp.

4. Composition according to claim 1, **characterised in that** the peptide according to general formula (I) corresponds to the sequence SEQ ID Ala-Val-Leu-Ala-Gly-NH2.

5. Composition according to claim 1, **characterised in that** the peptide according to general formula (I) corresponds to the sequence SEQ ID Ala-Val-Leu-Ala-Gly.

6. Composition according to any of claims 1 to 5, **characterised in that** the peptide according to general formula (I) has at least one functional group protected by a protecting group, this protecting group being either an acylation or an acetylation of the amino-terminus, or an amidation or an esterification of the carboxy-terminus, or both.

7. Composition according to any of the above claims, **characterised in that** the active ingredient is present in the composition at a concentration between approximately 0.0005 and 500 ppm, and preferentially at a concentration between 0.01 and 5 ppm in relation to the total weight of the composition.

8. Composition according to any of the above claims, **characterised in that** the active ingredient is previously solubilised in one or a plurality of cosmetically of pharmaceutically acceptable solvents, such as water, glycerol, ethanol, propylene glycol, butylene glycol, dipropylene glycol, ethoxylated or propoxylated diglycols, cyclic polyols, petroleum jelly, vegetable oil or any mixture of these solvents.

9. Composition according to any of the above claims, **characterised in that** it is presented in a form suitable for topical application.

10. Composition according to any of the above claims, **characterised in that** it further contains at least one further active agent chosen from vitamin C, vitamin E, plant polyphenolic extracts, collagen peptide marketed under the name Collaxyl®, and the active ingredient marketed under the name GP4G®.

11. Cosmetic use of a composition comprising an active ingredient as defined in any of claims 1 to 6.

12. Composition comprising an active ingredient as defined in any of claims 1 to 6, for use in a dermatological treatment.

13. Composition comprising an active ingredient as defined in any of claims 1 to 6 for increasing the intracellular ATP synthesis of skin cells.

14. Composition comprising an active ingredient as defined in any of claims 1 to 6 for increasing the activity or synthesis of trans-prenyl transferase enzyme and/or coenzyme Q10 in skin cells.

15. Compositions comprising an active ingredient as defined in any of claims 1 to 6 for protecting the skin and skin appendages against all types of external attacks.

16. Composition according to claim 15 for preventing or treating damage caused to the skin and skin appendages by UV rays.

17. Composition according to claim 15 for protecting the skin and skin appendages from oxidative damage.

18. Cosmetic use according to claim 11 for preventing or treating the skin signs of ageing and/or photoageing.
